# EUROPEAN PATENT APPLICATION

(11) **EP 2 641 607 A1**
(43) Date of publication of application: **25.09.2013**
(21) Application number: 12305332.4
(22) Date of filing: 21.03.2012
(51) Int. Cl.: A61K 35/74, A61K 31/739, C08B 37/00, C12P 19/04, C12R 1/89, A61P 29/00, A61P 11/00

(54) **A mixture of polar glycolipids for use in the treatment of pain and COPD**

(71) Applicant: SANOFI, 75008 Paris (FR)
(72) Inventor: Croci, Tiziano, 75008 Paris (FR); Guagnini, Fabio, 75008 Paris (FR); Zarini, Elena, 75008 Paris (FR)
(74) Representative: Martin-Touaux, Elsa

(57) **Abstract**

The present invention concerns a composition comprising a polar glycolipid obtainable from cells of a cyanobacterium *Oscillatoria* species (CyP), for use for the treatment and/or the prevention of pain and/or of Chronic Obstructive Pulmonary Disease (COPD).

## Description

The present invention concerns a composition comprising a polar glycolipid obtainable from cells of a cyanobacterium *Oscillatoria* species (CyP), for use for the treatment and/or the prevention of pain and/or of Chronic Obstructive Pulmonary Disease (COPD).

### BACKGROUND OF THE INVENTION

Cyanobacteria, also called blue-green algae, are a natural source of active ingredients with unique properties. Low molecular weight glycolipid extracts from cyanobacteria are well known to contain a lipid component belonging to the class of fatty acid esters of glycerol, for example monogalactosyl diacylglycerols (MGDG), digalactosyl diacylglycerols (DGDG), sulfoquinovosyl diacylglycerols (SQDG), phosphatidyl glycerol (PG), etc. Such low molecular weight extracts prepared from various cyanobacteria have shown various types of biological activities, for example anti-inflammatory properties as described, for instance, in the patent application EP1571203, and anti-tumoral properties as described in Shiraashi et al., 1993, Chem. Pharm. Bull., 41:1664-66. Additionally, sulpholipids extracted from cyanobacteria also showed anti-HIV activity (Gustafson, J. Natl. Cancer Instit., 1989, 81:1254-1258).

The patent applications WO 2010/010172 and WO 2010/094693 describe the preparation of a glycolipid fraction extracted from a freshwater cyanobacterium *Oscillatoria* species and comprising a polar glycolipid of high molecular weight (33 kDa) with a LPS-like structure, also named herein "VB3323". This highly purified preparation of polar glycolipids, which may be extracted from cells of the cyanobacterium *Oscillatoria Plankothotrix sp,* is characterized by the presence of at least one high-molecular-weight glycolipid species comprising one or more rhamnose units. Within such preparation, also characterized by a protein contamination lower than or equal to 2% of the total weight, in particular characterized by the absence of protein contaminants, as assessed for example by the Bradford method, and characterized by a nucleic acid contamination lower than or equal to 5%, in particular lower than or equal to 3% of the total weight, it was possible to detect an inhibitory activity toward ATP synthase (ATP-SX), resulting in reduction of extracellular ATP levels.

It has been shown in the patent applications WO 2010/010172 and WO 2010/094693 that this glycolipid extract has a potent anti-inflammatory activity. More precisely, it has been shown that the glycolipid extract has a potent TLR4 antagonist activity as it is able to antagonize the proinflammatory effects triggered by TLR4 receptor LPS agonists in human dendritic cells, such as the LPS from *E. coli* and *Salmonella abortus equi,* and to antagonise the pro-inflammatory effects of LPS from *Porphyromonas gingivalis.* Besides its action on TLR4, VB3323 has also been shown to partially inhibit the activity of cell surface ATP synthase with a resulting reduction of extracellular ATP levels.

Toll-Like receptors (TLRs) represent one of the main routes for recognition and entry of microbial pathogenic structures into cells responsible for immune recognition and stimulation. Moreover, recent studies have shown that TLRs play an important role not only in triggering an inflammatory response against potential pathogens, but also in the response activated by stressed or injured tissues. TLR4 is a key receptor involved in the amplification of inflammation. In particular, TLR4, which is known to be the receptor involved in the innate immune response to bacterial lipopolysaccharides (LPS), has been demonstrated to have an important role in the cellular signaling activated by endogenous molecules produced in response to stress and/or tissue damage. Some of these molecules are mediators released by necrotic cells such as the Heat Shock Proteins (HSPs) and High-Mobility Group protein B1 (HMGB1); others are products of the extracellular matrix such as biglycan, fragments of hyaluronic acid, and tenascin-C.

ATP is another important endogenous pro-inflammatory mediator whose down-regulation could have a relevant role in reducing the inflammatory state in synergy with TLR4 antagonist activity. The ATP synthase enzyme is expressed on many different cell types including hepatocytes, neurons, astrocytes, fibroblasts, immune cells, endothelial cells and is expressed at particularly high level on the plasma membrane of tumor cells.

Therefore, the patent applications WO 2010/010172 and WO 2010/094693 disclose the use of the glycolipid fraction extracted from *Oscillatoria Planktothrix sp.* for the treatment of a large number of various acute or chronic conditions caused by activation of a pro-inflammatory state.

### DESCRIPTION OF THE INVENTION

### Therapeutic use of the glycolipid mixture

The inventors of the present invention have shown that the glycolipid fraction from an *Oscillatoria* species, VB3323, alleviates pain in animal models of pain. In particular, they have shown that acute treatment with the glycolipid mixture potently and dose dependently reduced both thermal hyperalgesia and mechanical allodynia in a mouse model of chronic inflammation induced by Freund's adjuvant (Example 2). They have shown that chronic treatment with the glycolipid preparation as described herein reduced significantly mechanical allodynia (Example 3). Finally, they have shown that chronic treatment with the glycolipid preparation of the invention significantly reduced both thermal hyperalgesia and mechanical allodynia in a mouse model of neuropathic pain induced by sciatic nerve ligation (Example 5).

Therefore, a composition comprising such a glycolipid can advantageously be used for the treatment and/or the prevention of pain, in particular neuropathic pain.

Neuropathic pain results from damage to or dysfunction of the peripheral or central nervous system. Neuropathic pain may thus be divided into peripheral neuropathic pain, central neuropathic pain, or mixed (peripheral and central) neuropathic pain.

Neuropathic pain is often caused by nerve injury or diseases such as diabetes, acquired immunodeficiency syndrome or cancer, which damage peripheral nerves. Diagnosis is suggested by pain out of proportion to tissue injury, dysesthesia (e.g., burning, tingling), and signs of nerve injury detected during neurologic examination. One common symptom of neuropathic pain is tactile allodynia, which is characterized by painful responses to normally innocuous tactile stimuli.

Although neuropathic pain responds to opioids, treatment is often with adjuvant drugs (e.g. antidepressants, anticonvulsants, baclofen, topical drugs).

Pain can develop after injury to any level of the nervous system, peripheral or central; the sympathetic nervous system may be involved (causing sympathetically maintained pain). Specific syndromes include post-herpetic neuralgia, root avulsions, painful traumatic mononeuropathy, painful polyneuropathy (particularly due to diabetes), central pain syndromes (potentially caused by virtually any lesion at any level of the nervous system), postsurgical pain syndromes (e.g. post-mastectomy syndrome, post-thoracotomy syndrome, phantom pain), and complex regional pain syndrome (reflex sympathetic dystrophy and causalgia).

Therefore, the composition comprising the glycolipid of the invention can advantageously be used for the treatment and/or the prevention of peripheral neuropathic pain, central neuropathic pain, or mixed (peripheral and central) neuropathic pain. In particular, the composition comprising the glycolipid mixture of the invention can be used for the treatment and/or the prevention neuropathic pain caused by diabetes, acquired immunodeficiency syndrome or cancer.

The inventors have further shown that the glycolipid fraction from *Oscillatoria* inhibits release of TNF-a induced by E. Coli-LPS in human bronchus parenchyma tissue cultures (Example 7). Chronic Obstructive Pulmonary Disease (COPD) is a chronic inflammation caused by bacteria or bacterial products (LPS). The results obtained in human bronchus parenchyma tissue cultures indicates that VB3323 could inhibit *in vivo* inflammation induced by bacteria or bacterial products (LPS) and thereby enable for prevention and/or treatment of COPD.

Therefore, another aspect of the invention is the use of the composition comprising the glycolipid for the treatment and/or the prevention of COPD.

Chronic obstructive pulmonary disease (COPD) is partially reversible airflow limitation caused by an inflammatory response. Symptoms are productive cough and dyspnea that develop over years; common signs include decreased breath sounds, prolonged expiratory phase of respiration, and wheezing. Usual treatment includes bronchodilators, corticosteroids, and, when necessary, O₂ and antibiotics. About 50% of patients die within 10 year of diagnosis.

COPD comprises chronic obstructive bronchitis and emphysema. Many patients have features of both.

Chronic obstructive bronchitis is chronic bronchitis with airflow obstruction. Chronic bronchitis becomes chronic obstructive bronchitis if spirometric evidence of airflow obstruction develops. Chronic asthmatic bronchitis is a similar, overlapping condition characterized by chronic productive cough, wheezing, and partially reversible airflow obstruction; it occurs predominantly in smokers with a history of asthma. In some cases, the distinction between chronic obstructive bronchitis and chronic asthmatic bronchitis is unclear.

Emphysema is destruction of lung parenchyma leading to loss of elastic recoil and loss of alveolar septa and radial airway traction, which increases the tendency for airway collapse. Lung hyperinflation, airflow limitation, and air trapping follow. Airspaces enlarge and may eventually develop bullae.

The invention thus relates to a composition comprising a glycolipid, in particular a polar glycolipid, obtained or obtainable from cells of a cyanobacterium *Oscillatoria* species for use for the treatment and/or the prevention of pain and/or of Chronic Obstructive Pulmonary Disease (COPD).

The invention also relates to a method of treatment and/or prevention of pain and/or of COPD, said method comprising the step of administering the composition comprising a mixture of polar glycolipids as described herein to a subject in need thereof. The composition is administered in particular in a therapeutically effective amount.

The composition may comprise a pharmaceutically acceptable excipient, diluent and/or other stabilizer and may be administered through any suitable route. Particular routes of administration include oral, intra-venous (i.v.) or topical administration.

A composition comprising a cyanobacterial polar glycolipid prepared as described herein, showing a level of protein contamination below or equal to 2% of the total weight, in particular showing an absence of protein contamination, and a level of nucleic acid contamination below or equal to 5%, in particular to 3% of the total weight, are not cytotoxic for mammalian cells as assessed in cytotoxic assays in cell lines as well as in PBMC isolated from human blood. The lack of cytotoxicity of the composition has been tested in cellular systems *in vitro* up to a concentration (100 µg/ml), that is 100-fold higher than the minimum effective concentration. Its use for the treatment and/or the prevention of pain and/or of COPD while ensuring safety for the patient can therefore be envisioned.

Moreover, the inventors have shown that VB3323 chronic treatment does not induce any significant body weight change and organ alteration in a mouse model of chronic inflammation induced by Freund's adjuvant (Example 4), and reduces body weight loss induced by chronic pain in a mouse model of neuropathic pain induced by sciatic nerve ligation (Example 6). These data further support the potential use of the polar glycolipids mixture, as a therapeutic compound for the treatment and/or the prevention of neuropathic pain and/or of COPD.

The subject may be a human or a non-human mammal, such as a feline, a canine, a rodent, a primate, a bovine, an ovine or a horse. The subject is preferably a human, i.e. a child, a woman or man.

As used herein, the terms "treatment" or "treating" denotes reversing, alleviating, inhibiting the progress of the disorder or condition to which such term applies, or one or more symptoms of such disorder or condition. "Prevention" or "preventing" means preventing occurrence of the disorder or condition to which such term applies, or one or more symptoms of such disorder or condition.

A therapeutically effective amount of the polar glycolipid from *Oscillatoria* may be readily determined by the one skilled in the art. It may depend for instance on the age, the body weight, the condition, etc of the subject. Typically also, a therapeutically effective daily dose of the polar glycolipid from *Oscillatoria* may be comprised between 0.01 and 50 mg, more preferably between 0.1 and 5 mg or even more preferably between 0.3 and 3 mg.

### Characteristics of the composition comprising the polar glycolipid

The patent applications WO 2010/010172 and WO 2010/094693 have shown that a highly purified preparation of polar glycolipids, obtainable or obtained by extraction from the cells of the cyanobacterium *Oscillatoria* species deposited at the Culture Collection of Algae and Protozoa (CCAP, Scotland, United Kingdom) by Bluegreen Biotech s.r.l., via Massena 15, 20125 Milano, Italy, on July 9, 2008, under accession number No. 1459/45, is characterized by the presence of at least one high-molecular-weight glycolipid species comprising one or more rhamnose units.

Accordingly, the composition comprising a glycolipid to be used in the frame of the invention may be obtained or is obtainable from cells of a cyanobacterium *Oscillatoria* species, such as e.g. a cyanobacterium *Oscillatoria Planktothrix* species. Such a preparation of polar glycolipids is sometimes referred to as "CyP" in the scientific literature.

Said cyanobacterium *Oscillatoria* species may for example be any *Oscillatoria* species deposited at the Culture Collection of Algae and Protozoa (CCAP), such as e.g. those strains deposited under accession numbers No. 1459/45, No. 1459/43, or No. 1459/44.

In a specific embodiment, said *Oscillatoria* species is the species deposited at the Culture Collection of Algae and Protozoa (CCAP) under accession numbers No. 1459/45.

In another specific embodiment, said cyanobacterium *Oscillatoria* species is a freshwater cyanobacterium. In particular, the cyanobacterium *Oscillatoria* species may be a cyanobacterium present in the in the Lake Varese, Italy. For instance, said cyanobacterium may be the *Oscillatoria Planktothrix* FP1 species, as described in Pomati et al., J. Phycol. 2000;36:553-562, Macagno et al., J Exp Med. 2006;203(6):1481-92, Jemmett et al., Infect Immun. 2008;76(7):3156-63, and Thorgersen et al., Mol Immunol. 2008;45(13):3553-7.

The glycolipid according to the invention is a polar glycolipid. In particular, the polar glycolipid comprised in the composition may be a lipopolysaccharide (LPS).

Said composition comprising a polar glycolipid may for instance be a fraction, an extract, a mixture of glycolipids, a preparation, and/or a pharmaceutical composition.

Methods which may be used for obtaining such a composition comprising a polar glycolipid from cells of a cyanobacterium *Oscillatoria* species (CyP) are well known by the skilled in the art and are for instance described in Yi et al., Analyst. 2000;125(4):651-6, Macagno et al., J Exp Med. 2006;203(6):1481-92, Jemmett et al., Infect Immun. 2008;76(7):3156-63, and Thorgersen et al., Mol Immunol. 2008;45(13):3553-7.

In a specific embodiment, the polar glycolipid may be extracted from a cyanobacterium *Oscillatoria* species.

In particular, the composition comprising the polar glycolipid may be obtainable from a cyanobacterium *Oscillatoria* species by a first method comprising:
a) providing said cyanobacterium;
b) extracting the polar glycolipid from said cyanobacterium;
c) precipitating the polar glycolipid from step (b); and
d) purifying the extracted and precipitated polar glycolipid from step (c).

Preferably, step (b) may comprise the treatment with a denaturing solution comprising a chaotropic agent and an aprotic organic solvent such as chloroform.

Also preferably, the method allowing obtaining the composition comprising the polar glycolipid of the invention may comprise the steps of:
i) providing a concentrate of the cyanobacterium, preferably a freeze-dried concentrate obtained after collecting the cyanobacterium from a culture medium by centrifugation;
ii) suspending said concentrate in a solution;
iii) optionally making a treatment with microwaves and/or a liquid/solid separation, preferably by centrifugating and collecting the liquid phase (termed supernatant);
iv) mixing the suspension of cyanobacteria or the supernatant with a denaturing solution comprising a chaotropic agent and an aprotic organic solvent such as chloroform;
v) incubating the solution obtained at step (iv);
vi) making a liquid/liquid separation, preferably by centrifugating and collecting the aqueous liquid phase;
vii) precipitation of the glycolipid fraction, preferably by addition of a salt and of an organic solvent such as acetone to the supernatant;
viii) resuspending the precipitate in an aqueous solution;
ix) optionally treating the solution obtained at (viii) with an organic solvent such as isopropanol, centrifugating, and collecting the liquid phase preferably by centrifugation; and
x) passage through an ion exchange column and collection of the fraction comprising the polar glycolipid.

In another specific embodiment, the polar glycolipid can be prepared from a culture of *Oscillatoria sp.* as described the patent application WO 2010/094693. In this second method, the bacterial pellet is first treated with denaturing agents, such as a mixture of organic solvents and chaotropic agents, for example a mixture of phenol and guanidinium thiocyanate, and with deproteinizing agents. The process is further characterized by treatment with nucleases until reaching a level of nucleic acid contamination lower than or equal to 5% of the total weight, preferably lower than or equal to 3%, even more preferably lower than or equal to 2%, and by a step of molecular separation on a device with a cutoff of 30 KDa, followed by recovery of the higher molecular weight fraction. Preferably, the composition comprising the mixture of polar glycolipids shows a level of protein contamination below or equal to 2% of the total weight. In particular said composition is devoid of protein contaminants.

In particular, the composition comprising the polar glycolipid may be obtained or is obtainable from a cyanobacterium *Oscillatoria* species by a method comprising extraction with denaturing chaotropic agents, treatment of the extract with nucleases until reaching a level of nucleic acid contamination lower than or equal to 5%, e.g. lower than or equal to 3% of the total weight, molecular separation on a device with a cutoff of 30 KDa, followed by recovery of the higher molecular weight fraction.

More precisely, the composition comprising the polar glycolipid obtained from a culture of *Oscillatoria sp.* may for instance be prepared as follows.

Cyanobacteria are grown in culture medium, for example BG-1 1 medium (Sigma-Aldrich, Cat No. C3061), at a temperature of 25°C and under cool-white light at a light intensity of 5 umol.m⁻².sec⁻¹ and in a continuous 24-hour light regimen. Upon reaching the stationary growth phase, the cyanobacterial culture is sedimented, for example by centrifugation (pelletting); the sediment (or pellet) may be frozen or freeze-dried prior to extraction. After thawing or rehydration (in the case of freeze-drying), the sediment is processed through the following steps:
a) the pellet is resuspended by dilution preferably with a volume of water or aqueous solvent comprised between 1 :1 and 1 :2;
b) to obtain a cell extract containing the glycolipid mixture (also termed supernatant), the resuspended pellet is mixed, as described above, with an appropriate volume of solution containing denaturing agents, for example as described in Chomczynski P. and Mackey (Biotechniques, 1995, 19(6):942-5), preferably comprising a reagent based on a polar protic organic solvent, preferably phenol, and a chaotropic agent (such guanidinium thiocyanate), as for instance the Trireagent® (Sigma cat. N T3934) or similar reagents such as Trizol® (Invitrogen), and an aprotic organic solvent such as chloroform, at a ratio of about 1 volume of aqueous suspension of cyanobacteria, 2-4 volumes of extraction solution, preferably about 3 volumes, and about 0.5-1 volume of chloroform;
c) the cell extract is incubated for at least 5 minutes, more preferably for a time length of at least 10 minutes but not exceeding 60 minutes, at room temperature;
d) centrifugation, preferably at approximately 2000xg, and the supernatant (aqueous phase) containing the polar glycolipid fraction is collected. Such supernatant can be measured, for example, by electrophoresis and silver-staining or by biological activity assays, such as inhibition of TNF-alpha production in the monocyte/macrophage derived THP1 cell line in presence of LPS, or the binding to ATP synthase (ATP-SX) or the inhibition of its activity. The pellet obtained by centrifugation in d) can be re-extracted by adding water or an aqueous buffer (in an amount approximately equal to the volume collected), followed by re-centrifugation of the sample. This second supernatant is combined with the previous supernatant, and the pooled sample is then subjected to further steps involving:
e) precipitation by addition of salt, e.g. sodium acetate (5-20 mM final) and of an organic solvent, preferably acetone, preferably also in an amount of about 2 volumes, followed by centrifugation, under the same conditions as above, and by pellet washing with ethanol diluted in water, e.g. 70% ethanol, at least once and preferably twice,
f) resuspension of the pellet in a preferably buffered aqueous solution, for instance 50 mM TRIS,
g) enzymatic treatment of nucleic acid contaminants carried out by use of endo- and/or exo-nucleases (e.g. DNase and RNase), followed by enzymatic treatment of protein contaminants by digestion with a protease, e.g. proteinase K, preferably at 100 µg/ml for sufficient time (at least 1 hour at 37°C).

After enzymatic digestion, the sample is again centrifuged, the supernatant is collected and further precipitated by addition of salt (e.g. sodium acetate, about 10 mM final) and of an appropriate volume (preferably 2 volumes) of organic solvent, preferably acetone, centrifuged again, followed by resuspension of the pellet in water or aqueous solution containing at least one surfactant agent, preferably deoxycholate (DOC), and also preferably containing a cationic surfactant such as tetraethylammonium; the material is again extracted with the (denaturing) extraction solution described in section b), and then is further precipitated with sodium acetate/acetone as described above, washed with 70% ethanol, resuspended in water or in aqueous solution, and subjected to molecular separation by use of a filter (or other suitable device) with a cut off of 30 kDa, thus eliminating the material that passes through the filter and recovering the higher molecular weight glycolipid fraction in the retentate, in water or buffered aqueous solution, with a level of nucleic acid contamination lower than 5% of the total weight.

The composition comprising the polar glycolipid may thus be obtained or is obtainable from cells of a cyanobacterium *Oscillatoria sp* by a method comprising with denaturing chaotropic agents, wherein the extraction step is performed as described in following step b), nuclease treatment is performed as described in following step g), molecular separation is performed as described in following step k) and comprises the further following steps a), c)-f) e h)-j) and I)-m):
a) resuspension of a concentrate of the cyanobacterium *Oscillatoria sp.* (No. 1459/45), optionally also freeze-dried, with an aqueous solution in a volume ratio preferably comprised between 1:1 and 1:2;
b) mixing the suspension of cyanobacteria with 2-4 volumes, preferably about 3, of a denaturing solution comprising a chaotropic agent, a polar protic organic solvent, preferably phenol, and an aprotic organic solvent such as chloroform,
c) incubation for a time shorter than 60 minutes;
d) centrifugation and collection of the liquid phase, termed supernatant;
e) precipitation of the glycolipid fraction by addition of a salt and an organic solvent, preferably acetone, to the supernatant and washing the precipitate (or pellet) with water-diluted ethanol;
f) resuspension of the precipitate in an aqueous solution, preferably buffered;
g) treatment with nucleases, preferably endo- and/or exo-nucleases and subsequently with a protease, preferably proteinase K;
h) precipitation of the glycolipid phase by addition of a salt and an organic solvent, preferably acetone;
i) optional step of pellet washing with water-diluted ethanol and resuspension in aqueous solution comprising ionic surfactants, preferably sodium deoxycholate, and a quaternary ammonium salt;
j) re-extraction from the aqueous solution as described in steps b)-f);
k) molecular separation on a device with a cutoff of 30 KDa;
l) recovery of the high molecular weight polar glycolipid fraction with water or buffered aqueous solution and assessment of the level of nucleic acid or protein contamination;
m) recovery and use of the fraction having less than 5%, e.g. less than 3% nucleic acid contamination of the total weight.

The activity of the so obtained glycolipid fraction can be measured by biological tests based on inhibition of the pro-inflammatory activity induced by LPS (from P. gingivalis or E. coli) in cells that produce cytokines such as IL-6, IL-8 TNF-alpha and/or IL-1beta, as for example THP1, and/or by biochemical assays, as for example electrophoresis. Alternatively, the activity of the so obtained glycolipid fraction may for instance be evaluated by measuring its inhibiting effect on TLR4-mediated NF-kB activation induced by LPS in cells engineered to stably co-express a human TLR-4 gene and a NF-kB-inducible SEAP reporter gene. The glycolipid fraction activity may also be observed by measuring its inhibiting effect on TNF-α release following LPS incubation on human lung parenchyma, as in Example 7.

WO 2010/094693 further describes the analytic characteristics of an extract comprising the glycolipid according to the invention, obtained by the method described hereabove.

The analysis of sugars of the polar glycolipid fraction indicates that, within the saccharide component (given as 100%), rhamnose is preferably 20-40%, glucose (Glc) is 20-40%, xylose (Xyl) is 5-10%, mannose (Man) is 3-5%, galactose (Gal) is 3-5%, glucosamine (GlcN) is 0.5-1 %, galacturonic acid (GalA) is 1-6%, where these sugars can be simple sugars or more preferably they are complex sugars and/or sugar derivatives. Qualitative and quantitative analysis of the saccharide residues can be done by GC-MS analysis of acetylated methyl glycosides after hydrolysis of the glycolipid mixture with methanol-hydrochloric acid (HCl 1 M/MeOH) for 20 hours at 80°C, extraction in hexane and acetylation with pyridine and acetic anhydride and/or by analysis of alditol acetates obtained after treatment with 2M trifluoroacetic acid for 1 hour at 120°C, followed by treatment for 1 hour with sodium borohydride and subsequent acetylation with pyridine and acetic anhydride.

Analytical analysis of the major glycolipid component of the glycolipid mixture has shown the following relative composition (percentages in weight / weight) :
- saccharides : rhamnose 51%, galacturonic acid 15%, mannose 11%, galactose 11%, xylose 4%, glucose 4%, glucosamine 4%;and
- fatty acids: octadecanoic acid, (C 18:0, 55%); hexadecanoic acid and 3-hydroxyhexadecanoic acid (C16:0 + C16(3OH)), 35%); 3-hydroxyundecanoic acid (C15(3OH)), 10%).

The elementary analysis showed the occurrence of carbon 41%, hydrogen 6,5%, and nitrogen 4%.

Therefore, the polar glycolipid may be **characterized in that** it comprise at least one unit of rhamnose or its derivatives, and by molecular weights greater than 30 kDa, preferably between 30 and 40 kDa, still preferably 32 to 34 kDa (about 33 kDa), which can be determined, for example, by DOC-PAGE followed by staining with silver salts.

In particular, the composition of the invention may comprise a mixture of polar glycolipids comprising stearic acid (C18:0, octadecanoic) and/or palmitic acid (C16:0, hexadecanoic) as fatty acids of the major glycolipid component, wherein at least one of them is associated with a saccharide comprising at least one unit of rhamnose or its derivatives.

Such fatty acids exist preferably in their saturated form and they are preferably present in amounts respectively comprising (where 100 is the total lipid fraction) 50%-80% stearic acid and/or 15%-40% palmitic acid, each in respect to the total lipid fraction of the glycolipid mixture, and even more preferably such fatty acids are present in amounts comprised between 65-75% and 20-32%, respectively. Other fatty acids chains may be present in amounts not exceeding 15% and preferably comprise Lauroleic acid (or dodecenoic acid (C12: 1)) or 3-hydroxyundecanoic acid in amounts not exceeding 10%, preferably not more than 5% of the lipid fraction. The analysis of fatty acids can be carried out by GC-MS after hydrolysis of the compound with methanol-hydrochloric acid (HCl 1M/MeOH) for 20 hours at 80°C and extraction as methyl esters in the hexane phase, as described in WO 2010/094693.

Preferably, the glycolipid mixture is devoid of phospholipids and free lipids. Moreover, the glycolipid mixture may also be devoid of low molecular weight glycolipids such as fatty acid derivatives of glycerol (monogalactosyl diacylglycerols (MGDG), digalactosyl diacylglycerols (DGDG), sulfoquinovosyl diacylglycerols (SQDG), which are typical of cyanobacterial thylakoid membranes, have a molecular weight generally lower than 1 kDa.

### Pharmaceutical compositions

The complete water solubility of the compound makes possible a therapeutic use in pharmaceutical compositions with aqueous excipients and diluents, which are well known to the expert in the field, especially with respect to liquid formulations. The glycolipid extract can be diluted both in water and in an aprotic solvent such as DMSO and is stable in aqueous solution. Moreover, it is also heat stable.

Thus, the glycolipid of the invention may be formulated in a pharmaceutical composition. Compositions according to the invention comprise the glycolipid extract from *Oscillatoria sp.,* preferably in concentrations comprised between 0.1 and 100 µg/ml, more preferably in concentrations comprised between 1 and 50 µg/ml or even more preferably in concentrations comprised between 4 and 20 µg/ml.

For oral, intra-venous (i.v.) and topical administration, the daily glycolipid extract dose to be administered to a patient is preferably comprised between 0.01 and 50 mg, more preferably comprised between 0.1 and 5 mg or even more preferably comprised between 0.3 and 3 mg.

Moreover, in one specific aspect, these compositions comprise pharmaceutically acceptable excipients, diluents and/or other active ingredients and stabilizers. In particular, the glycolipid extract can be associated with a pharmaceutically acceptable carrier that is suitable for oral, intra-venous (i.v.) or topical administration.

As used herein, the term "pharmaceutically acceptable carrier" includes solvents, dispersion media, coatings, isotonic and absorption delaying agents and the like, that do not produce an adverse or other untoward reaction when administered to an animal, or a human, as appropriate.

Preferred oral dosage forms include solid forms, adhesive tablets, biofilms or creams, ointments and pastes. A preferred form is a tablet, e.g. as those described in U.S. patent No. 5,244,663.

Carriers may further include, but are not limited to disintegrants, binders, lubricants, flavoring, colorants, preservatives. Suitable disintegrants include dry starch, calcium carbonate, polyoxyethylene sorbitan fatty acid esters, sodium lauryl sulfate, stearic monoglyceride, lactose, as well as cross-linked polyvinylpyrrolidones, such as crospovidone (e.g., Polyplasdone™. XL, which may be obtained from GAF), cross-linked carboxylic methylcelluloses, such as croscarmelose (e.g., Ac-di-sol™, which may be obtained from FMC), alginic acid, and sodium carboxymethyl starches (e.g., Explotab™, which may be obtained from Edward Medell Co., Inc.), methylcellulose, agar bentonite and alginic acid. Binders, if used, are those that enhance adhesion. Examples of such binders include, but are not limited to, starch, gelatin and sugars such as sucrose, dextrose, molasses, and lactose. Preferred lubricants are stearates and stearic acid.

Lactose, mannitol, and croscarmelose are preferred excipients.

Additional components that may be incorporated into the dosage forms are known, or will be apparent, to those skilled in this art; for example, see Remington: The Science and Practice of Pharmacy, 19th edition (Mack Publishing, 1995).

In a specific embodiment the pharmaceutical composition is in a liquid form.

For the preparation of aqueous suspensions, one may employ various sweetening or flavouring agents, colouring matter and, if so desired, emulsifying and/or suspending agents, together with such diluents as water, ethanol, propylene glycol, glycerine and various combinations thereof. Glycerine is preferred.

According to a further aspect, the mixture is also suitable for preparation of compositions for veterinary use, with the same therapeutic purpose described above.

### BRIEF DESCRIPTION OF THE FIGURES

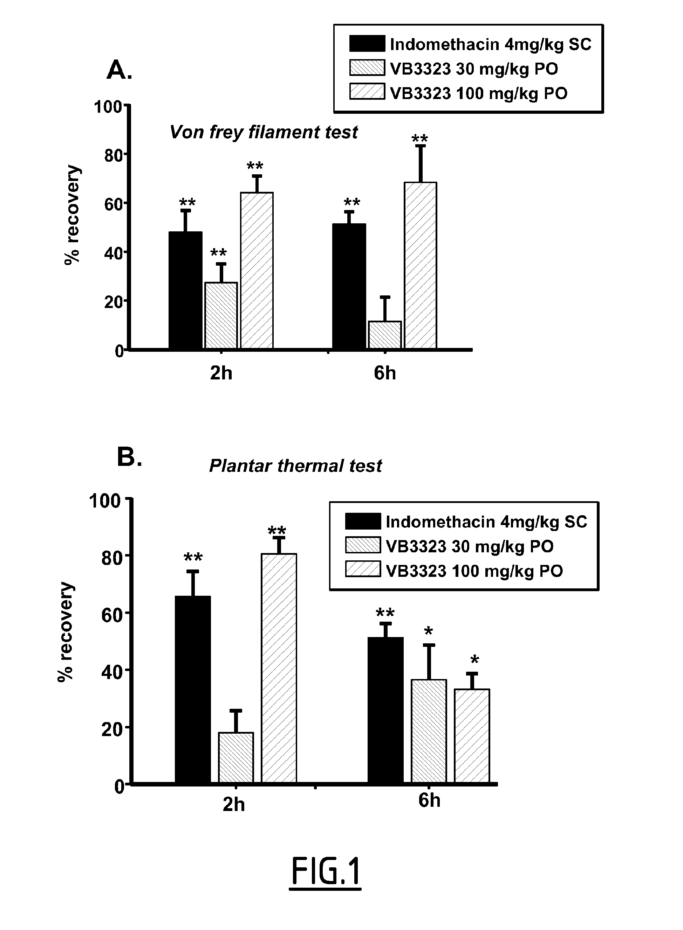

Figure 1: Effect of VB3323 acute treatment on mechanical allodynia and thermal hyperalgesia in Freund complete adjuvant model

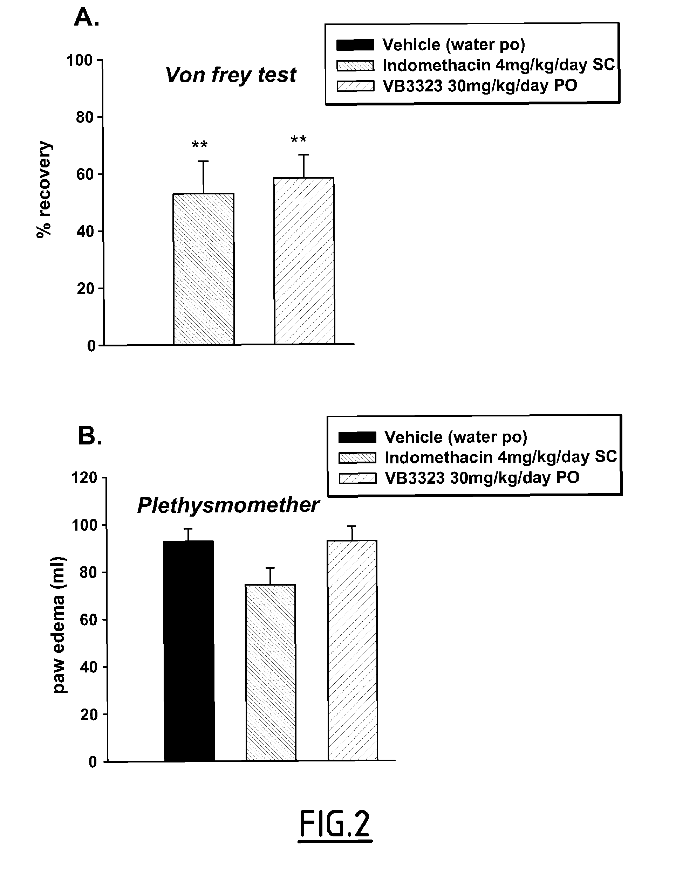

Figure 2: Effect of VB3323 oral therapeutic chronic treatment on mechanical allodynia and paw edema in FCA model

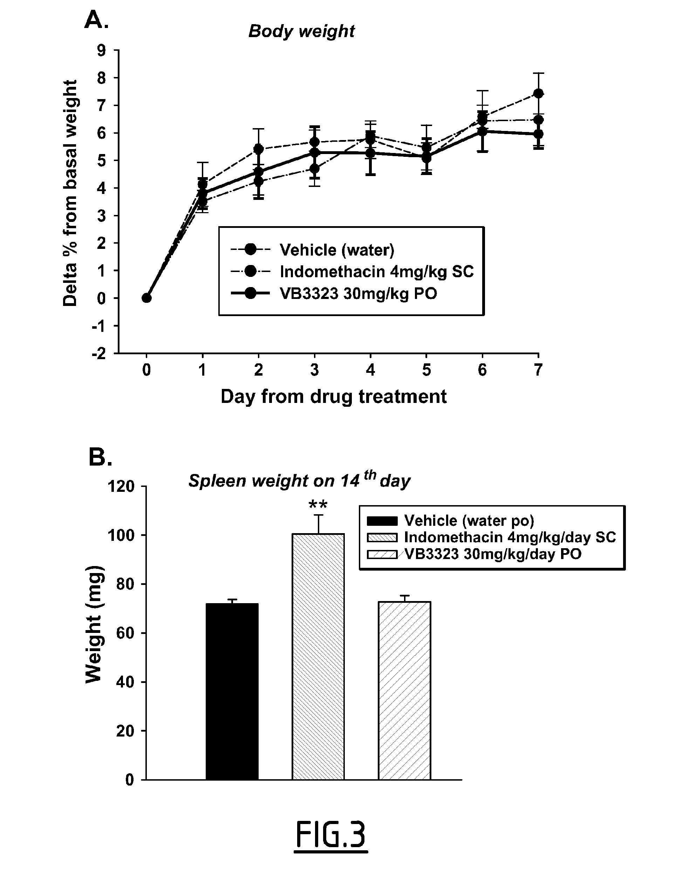

Figure 3: Effect of VB3323 oral therapeutic chronic treatment on mice body weight and organs gross morphology evaluations

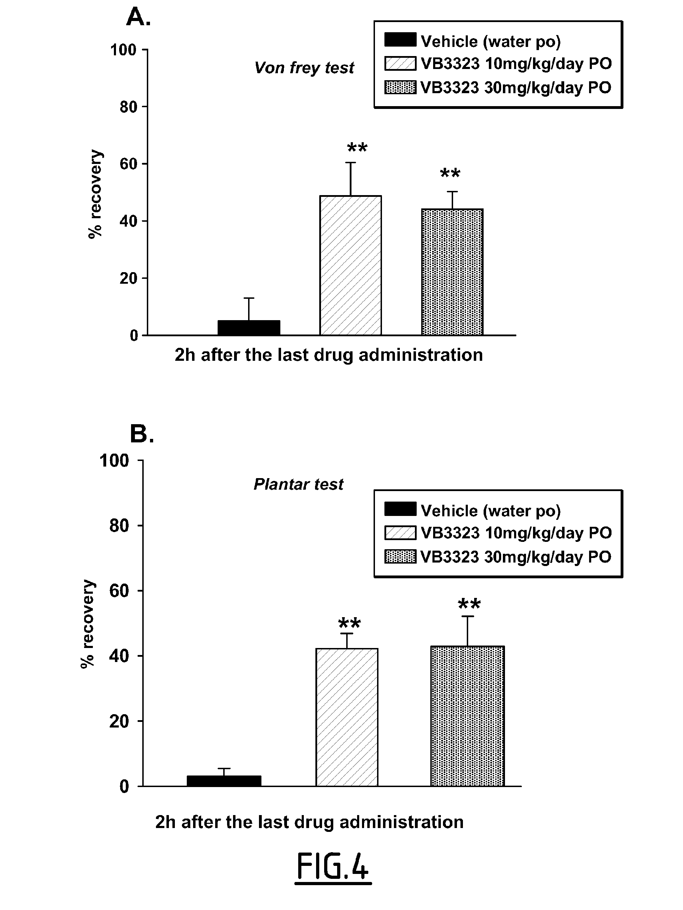

Figure 4: Effect of VB3323 chronic treatment on mechanical allodynia and thermal hyperalgesia in sciatic nerve ligation model

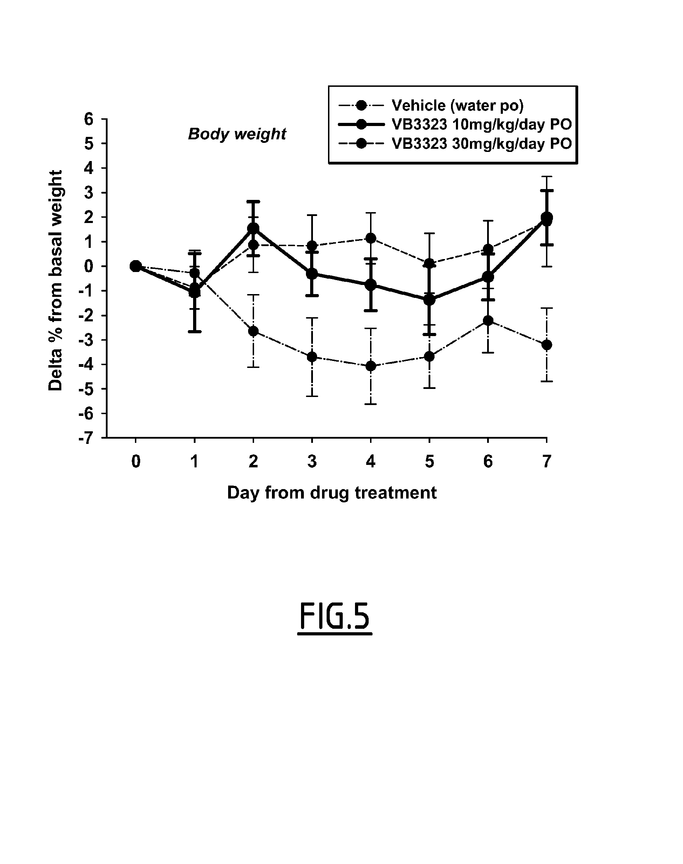

Figure 5: Effect of VB3323 chronic treatment on mice body weight

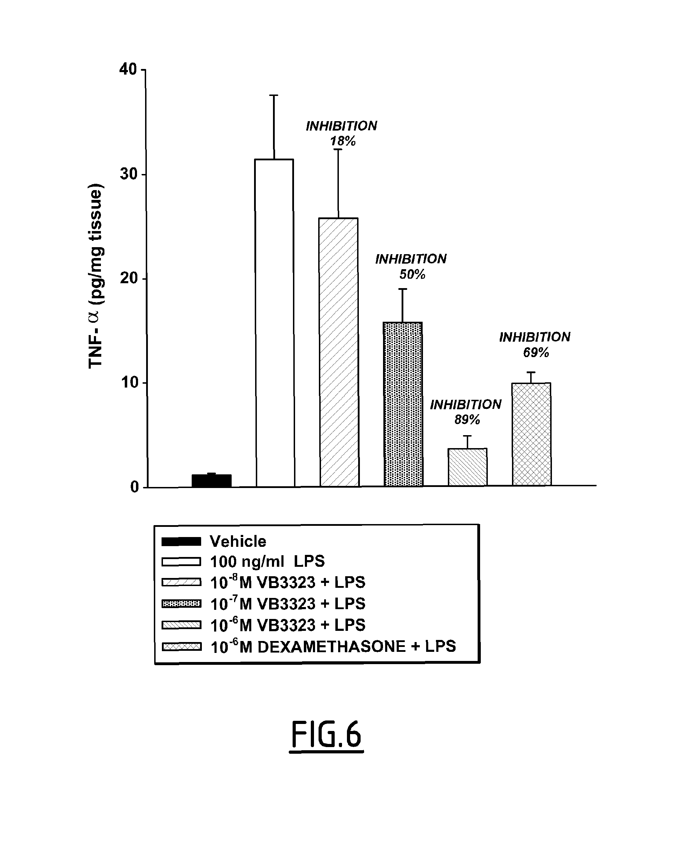

Figure 6: Effect of VB3323 on TNFα release during E. Coli-LPS incubation
Figure 7: NMR profile of three different batches of VB3323

### EXAMPLES

### Example 1: Materials and Methods

### VB3323

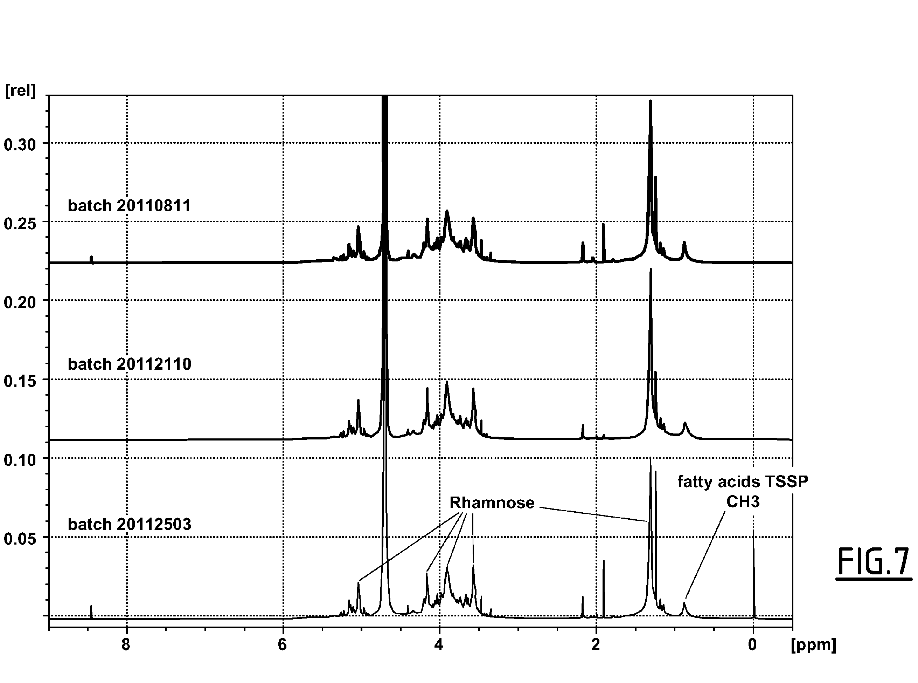

VB3323 was obtained from Bluegreen Biotech (Milano, Italy). VB3323 corresponds to a composition that is a glycolipidic fraction comprising a lipopolysaccharide (LPS), said glycolipidic fraction being extracted from the Oscillatoria strain deposited at the Culture Collection of Algae and Protozoa (CCAP) under accession number No. 1459/45. The NMR profile of three different batches of VB3323 is shown on Figure 7.

### Models used for pain and COPD

The animal models used in Examples 1 to 6 are well-known in the art. The experiments were carried out using standard procedures. This sciatic nerve ligation mouse model and the nociceptive test are further described below. The cellular models used in Examples 7 and 8 are described in the corresponding examples.

### Sciatic nerve ligation

C57BU6N, Crl BR mice (wild type) weighing 28±3 g, were anesthetized with sodium pentobarbital (65 mg/kg, i.p.) and submitted to surgical procedure. Briefly, the common sciatic nerve was exposed at the level of the mid thigh and, proximal to the sciatic nerves trifurcation, four ligatures were loosely tied around it with about 1 mm spacing so that the epineural circulation was preserved. In mice the ligatures were tied until a brief twitch was seen in the respective hindlimb. Sham animals (sciatic exposure without ligation) were used as controls

### Nociceptive tests

Thermal hyperalgesia was evaluated by recording the paw withdrawal latencies to a radiant thermal stimulus according to Hargreaves' method (plantar test), using a specific instrument (Ugo Basile, Varese, Italy) (Hargreaves et al.,1988). Animals were placed in a clear acrylic box on a glass platform and allowed to acclimatize. A constant-intensity radiant-heat source was applied, through the platform, to the plantar surface of the hind-paw. The response to the stimulus was recorded by a photocell detecting withdrawal of the paw, and expressing the latency in seconds. Thermal hyperalgesia was evaluated in the injected and contralateral hind-paw before, 7 and 14 days (24h after the last drug treatment) following CFA injection or at different time in the CCI model.

Mechanical allodynia was assessed according to the method described by von Frey (Villetti et al., 2003), using an instrument that measures the reaction to a mechanical stimulus (Plantar Aesthesiometer, Ugo Basile, Italy). Briefly, mice were placed on a metal mesh table and, after acclimatization, an electronic needle was pushed against the plantar foot surface with ascending mechanical force (50 g over a 20-second period) until the mice withdrew the paw. Mechanical allodynia was evaluated by measuring the withdrawal threshold in grams in the injected and contralateral hind-paw before, 7 and 14 days (24h after the last drug treatment) following FCA injection or at different time for CCI model.

### Example 2: VB3323 potently and dose dependently reduces both thermal hyperalgesia and mechanical allodynia in FCA treated mice

Fourteen days after intraplantar Freund Complete Adjuvant (FCA) injection, mice showed a significant decrease (about 50%) in both thermal withdrawal latency and mechanical allodynia threshold in the FCA treated paw.

Oral VB3323 (30 and 100 mg/kg), significantly and dose dependently, inhibited mechanical allodynia at 2 hours (28 and 67% of recovery) and 6 hours (15% and 69% recovery, respectively) after its administration on the 14^{th} day after FCA administration. In the same experiment, VB3323 showed an activity on thermal hyperalgesia at 2 hours (19 and 80% recovery) and 6 hours (39 and 38% recovery). Similarly to 100mg/kg dose, VB3323 at 10 mg/kg ip was able to reduce both mechanical allodynia and thermal hyperalgesia.

### Example 3: VB3323 at 30mg/kg chronic treatment reduces significantly mechanical allodynia

In mice treated with intraplantar Freund Complete Adjuvant (FCA), chronic oral treatment with VB3323 (30mg/kg for 7 days) reduced significantly mechanical allodynia (59% recovery) on the 14^{th} day after FCA administration. This effect was assessed 24h after the last VB3323 treatment.

### Example 4: VB3323 chronic treatment does not induce any significant body weight changes and organ alterations by gross morphology evaluation

Mice body weight was monitored daily starting from VB3323 treatment. Organ alterations by gross morphology evaluation and spleen weight were evaluated in animal killed 24 hours after the last VB3323 treatment.

VB3323 chronic treatment did not induce any alterations of the parameters above mentioned.

### Example 5: VB3323 significantly reduces mechanical allodynia and thermal hyperalgesia

Fourteen days after the chronic constriction injury of sciatic nerve, a model of chronic neuropathic pain, mice showed a significant decrease (about 50%) in both thermal withdrawal latency and mechanical allodynia threshold in the ligated paw. Oral VB3323 (10 and 30 mg/kg/daily for 8 days) significantly inhibited allodynia (45 and 50% recovery) and thermal hyperalgesia (42 and 43% recovery) at 2h from its last administration.

In this model VB3323 had no effect after a single administration on allodynia and it had a scant effect on thermal hyperalgesia. The activity of VB3323 is marked after repeated administration suggesting an important therapeutic activity.

### Example 6: VB3323 chronic treatment reduces body weight loss induced by chronic pain

Neuropathic pain caused by sciatic nerve ligation induced a reduction of mice body weight (delta % from basal body weight about 4).

VB3323 chronic treatment reduced (10 and 30 mg/kg/daily for 8 days) body weight loss induced by chronic pain.

Taken together, the results of Examples 1 to 6 demonstrate that VB3323 allows treating pain, and in particular neuropathic and/or chronic pain.

### Example 7: VB3323 inhibits LPS-induced TNFα release in human bronchus parenchyma

Human parenchymal lung tissue was obtained from patients undergoing resection for carcinoma at San Gerardo Hospital, Monza, Italy. Patients had not received radiotherapy or chronic treatment with steroids, opioids or chemotherapy. All specimens of parenchymal tissue were obtained from sites distant from the tumour. The study was approved by the institutional ethics committee of San Gerardo hospital Monza Italy and all volunteers gave informed consent. Resected lung tissue was dissected free of tumour, large airways, pleura and visible blood vessels and finely chopped using dissecting scissors, into 2 mm fragments during several washes with Kreb's buffer ((nM composition: NaCl 118.4, KCI 4.7, CaCl2 2.5, KH2PO4 1.2, NaHCO3 25, glucose 11.7). Six explants (total weight approx. 30 mg) were incubated in culture bioreactor (Synthecon/NASA USA) with RPMI-1640 medium containing 1% penicillin, 1% streptomycin, and 1% gentamycin at 37°C in 5% carbon dioxide/air for 16 hours. Tissue was then either stimulated with 100 ng/ml LPS (Sigma-Aldrich) or maintained in cell culture media alone for 4 hours. For neutralization of TNF, tissue was incubated with 0.01, 0.1, or 1 µM of VB3323 for 1 hr prior to stimulation with 100 ng/ml LPS. Lung tissue fragments and supernatant were harvested and both were stored at -80°C until analysis. The tissue fragments were weighed to determine total tissue weight to normalize the levels of released cytokines. The levels of TNFalpha in the supernatant were measured by enzyme-linked immunosorbent assay (ELISA) and the concentration corrected for tissue weight. Human TNF-specific ELISA kits (limit of detection of 0.2 pg/mg of tissue ) were purchased from Pirce, the manufacturer's protocol was followed for each ELISA.

VB3323, concentration dependently (0.01, 0.1, or 1µM), inhibited the release of TNFalpha induced by E. Coli-LPS in human bronchus parenchyma tissue culture with a IC50 of 0.1 µM. VB3323 at 1µM was more potent (89%) in the inhibition of TNFalpha release in comparison with the reference standard Dexamethasone (69%) at the same concentration.

Dexamethasone is a drug that is used for treating COPD in humans. Here, the inventors have found that VB3323 is more potent than Dexamethasone. Therefore, VB3323 is a very promising drug for the treatment of COPD.

### Example 8: VB3323 inhibits LPS-induced TNFα release in mouse bronchus parenchyma

The animals (BALB/c male mice, 20-22g body weight) were sacrificed by cervical dislocation. The thoracic cage was opened cutting the ribs. Clamping the heart with surgical tweezers, lungs and heart were dissected from the thoracic cavity using surgical scissors and putted in cold Krebs solution (nM composition: NaCl 118.4, KCI 4.7, CaCl2 2.5, KH2PO4 1.2, NaHCO3 25, glucose 11.7). Large airways and visible blood vessels were dissected. The lungs were divided in fragments of 30mg (∼ 5 pieces/mouse) and finely chopped (six pieces of ∼ 2 mm3 each fragment) using a scalpel. Six explants (total weight 30mg) were incubated per well (2.0 cm2) of a 24 well plate with 1ml of RPMI-1640 medium (RPMI-1640 + antibiotic antimycotic solution 100x, Sigma-Aldrich) at 37°C in 5% carbon dioxide/air for 16 hours. Then the medium was replaced with fresh medium and the tissue was treated for 1 hour with compound. After one hour, the tissue was stimulated with 100 ng/ml LPS E. Coli 055:B5 (Sigma-Aldrich). 4 hour after LPS stimulation, supernatant was harvested and stored at -80°C until ELISA analysis. Three of six tissue explants were fixed in 10% formalin buffer for histological/immunohistochemical analysis. The other three tissue explants were put in RNA-later buffer overnight and then stored at -80°C.

Mice TNFspecific ELISA kits (limit of detection of 0.2 pg/mg of tissue) were purchased from Pirce, The manufacturer's protocol was followed for each ELISA.

VB3323, at 1µM inhibited the release of TNFalpha induced by E Coli LPS in mouse bronchus parenchyma tissue culture. With an activity of 68% similar to that of the reference standard Dexamethasone (67%) at the same concentration.

This experiment confirms that VB3323 is a very promising drug for the treatment of COPD.

## Claims

1. A composition comprising a polar glycolipid obtainable from cells of a cyanobacterium *Oscillatoria* species (CyP), for use for the treatment and/or the prevention of pain and/or of Chronic Obstructive Pulmonary Disease (COPD).

2. The composition for the use according to claim 1, wherein said polar glycolipid is a lipopolysaccharide (LPS).

3. The composition for the use according to claim 1 or 2, wherein said polar glycolipid is extracted from a cyanobacterium *Oscillatoria* species.

4. The composition for the use according to any one of claims 1 to 3, wherein said polar glycolipid is obtainable from a cyanobacterium *Oscillatoria* species by a method comprising:
a) providing said cyanobacterium;
b) extracting the polar glycolipid from said cyanobacterium;
c) precipitating the polar glycolipid of step (b); and
d) purifying the extracted and precipitated polar glycolipid of step (c).

5. The composition for the use according to claim 4, wherein step (b) comprises the treatment with a denaturing solution comprising a chaotropic agent and an aprotic organic solvent such as chloroform.

6. The composition for the use according to any one of claims 1 to 5, wherein said method comprises the steps of:
i) providing a concentrate of the cyanobacterium, preferably a freeze-dried concentrate obtained after collecting the cyanobacterium from a culture medium by centrifugation;
ii) suspending said concentrate in a solution;
iii) optionally making a treatment with microwaves and/or a liquid/solid separation, preferably by centrifugating and collecting the liquid phase (termed supernatant);
iv) mixing the suspension of cyanobacteria or the supernatant with a denaturing solution comprising a chaotropic agent and an aprotic organic solvent such as chloroform;
v) incubating the solution obtained at step (iv);
vi) making a liquid/liquid separation, preferably by centrifugating and collecting the aqueous liquid phase;
vii) precipitation of the glycolipid fraction, preferably by addition of a salt and of an organic solvent such as acetone to the supernatant;
viii) resuspending the precipitate in an aqueous solution;
ix) optionally treating the solution obtained at (viii) with an organic solvent such as isopropanol, centrifugating, and collecting the liquid phase preferably by centrifugation; and
x) passage through an ion exchange column and collection of the fraction comprising the polar glycolipid.

7. The composition for the use according to any one of claims 1 to 3, wherein said polar glycolipid is obtainable from a cyanobacterium *Oscillatoria* species by a method comprising extraction with denaturing chaotropic agents, treatment of the extract with nucleases until reaching a level of nucleic acid contamination lower than or equal to 5% of the total weight, molecular separation on a device with a cutoff of 30 KDa, followed by recovery of the higher molecular weight fraction.

8. The composition comprising a polar glycolipid for the use according to claim 7, which is obtainable from cells of the cyanobacterium *Oscillatoria* species by a method comprising extraction with denaturing chaotropic agents, wherein the extraction step is performed as described in following step b), nuclease treatment is performed as described in following step g), molecular separation is performed as described in following step k) and comprises the further following steps a), c)-f) e h)-j) and I)-m):
a) suspending a concentrate of the cyanobacterium *Oscillatoria* species, optionally freeze-dried, with an aqueous solution in a volume ratio preferably comprised between 1:1 and 1:2;
b) mixing the suspension of cyanobacteria with 2-4 volumes, preferably about 3, of a denaturing solution comprising a chaotropic agent, a polar protic organic solvent, preferably phenol, and an aprotic organic solvent such as chloroform,
c) incubating for a time shorter than 60 minutes;
d) centrifugating and collecting of the liquid phase, termed supernatant;
e) precipitation of the glycolipid fraction by addition of a salt and an organic solvent, preferably acetone, to the supernatant and washing the precipitate (or pellet) with water-diluted ethanol;
f) resuspension of the precipitate in an aqueous solution, preferably buffered;
g) treatment with nucleases, preferably endo- and/or exo-nucleases and subsequently with a protease, preferably proteinase K;
h) precipitation of the glycolipid phase by addition of a salt and an organic solvent, preferably acetone;
i) optionally pellet washing with water-diluted ethanol and resuspension in aqueous solution comprising ionic surfactants, preferably sodium deoxycholate, and a quaternary ammonium salt;
j) re-extraction from the aqueous solution as described in steps b)-f);
k) molecular separation on a device with a cutoff of 30 KDa;
l) recovery of the high molecular weight polar glycolipid fraction with water or buffered aqueous solution and assessment of the level of nucleic acid or protein contamination;
m) recovery and use of the fraction having less than 5% nucleic acid contamination of the total weight.

9. The composition for the use according to any one of claims 1 to 7, wherein said composition comprises a mixture of polar glycolipids comprising stearic acid (C18:0, octadecanoic) and/or palmitic acid (C16:0, hexadecanoic) as fatty acids of the major glycolipid component, wherein at least one of them is associated with a saccharide comprising at least one unit of rhamnose or its derivatives.

10. The composition for the use according to claim 9, wherein said mixture of polar glycolipids comprises 50%-80% stearic acid (C18:0, octadecanoic) and/or 15%-40% palmitic acid (C16:0, hexadecanoic) in respect to the total lipid fraction of the glycolipid mixture.

11. The composition for the use according to claim 9 or 10, wherein said mixture of polar glycolipids comprises 65-75% stearic acid (C18:0, octadecanoic) and/or 20-32% palmitic acid (C16:0, hexadecanoic) in respect to the total lipid fraction of the glycolipid mixture.

12. The composition for the use according to any one of claims 1 to 11, wherein said cyanobacterium *Oscillatoria* species is the *Oscillatoria* species deposited at the Culture Collection of Algae and Protozoa (CCAP) under accession number No. 1459/45.

13. The composition comprising polar glycolipid for the use according to any one of claims 1 to 12, for the treatment and/or the prevention of pain.

14. The composition comprising a polar glycolipid for the use according to claim 13, for the treatment and/or the prevention of neuropathic pain.

15. The composition comprising a polar glycolipid for the use according to claim 13, for the treatment and/or the prevention of peripheral neuropathic pain, central neuropathic pain, or mixed (peripheral and central) neuropathic pain.

16. The composition comprising a polar glycolipid for the use according to claim 13, for the treatment and/or the prevention neuropathic pain caused by diabetes, acquired immunodeficiency syndrome or cancer.

17. The composition comprising a polar glycolipid for the use according to any one of claims 1 to 12, for the treatment and/or the prevention of COPD.

18. The composition comprising a polar glycolipid for the use according to any one of claims 1 to 17, wherein said composition further comprises a pharmaceutically acceptable excipient, diluent and/or other stabilizer.

19. The composition comprising a polar glycolipid for the use according to any one of claims 1 to 18, wherein said composition is for oral, intra-venous (i.v.) or topical administration.
